# EUROPEAN PATENT APPLICATION

(11) **EP 4 520 264 A1**
(43) Date of publication of application: **12.03.2025**
(21) Application number: 23839409.2
(22) Date of filing: 20.06.2023
(51) Int. Cl.: A61B 5/11, A61B 5/107

(54) **WALKING STATE EVALUATION SYSTEM AND PROGRAM**

(30) Priority: 11.07.2022 JP 2022110999; 28.04.2023 JP 2023074058
(71) Applicant: Imasen Electric Industrial Co., Ltd., Inuyama-shi, Aichi 484-8507 (JP)
(72) Inventor: KIYOHARA, Takehiko, Inuyama-shi, Aichi 484-8507 (JP); MATSUMOTO, Takanari, Inuyama-shi, Aichi 484-8507 (JP); HIGUCHI, Makoto, Inuyama-shi, Aichi 484-8507 (JP); UEDA, Masaru, Inuyama-shi, Aichi 484-8507 (JP); FUKUSHIMA, Hiroshi, Inuyama-shi, Aichi 484-8507 (JP); HIDA, Toshihiko, Inuyama-shi, Aichi 484-8507 (JP); MIWA, Masaki, Inuyama-shi, Aichi 484-8507 (JP)
(74) Representative: Gille Hrabal Partnerschaftsgesellschaft mbB Patentanwälte
(86) International application number: PCT/JP2023/022720
(87) International publication number: WO 2024/014234

(57) **Abstract**

An information acquisition means, a condition evaluation means, and an evaluation output means are provided, in which the information acquisition means acquires subject walking information relevant to a walking condition of a subject, the condition evaluation means compares the walking information acquired by the information acquisition means with ideal walking information relevant to an ideal walking condition to evaluate the walking condition of the subject, the evaluation output means outputs an evaluation result by the condition evaluation means, ideal walking information configured of a plurality of parameters including a hip joint angle in walking according to the ideal walking condition is prepared for each hip joint angle over a predetermined range, as the ideal walking information, the information acquisition means acquires a plurality of parameters including a hip joint angle in walking according to the subject, as the subject walking information, and the condition evaluation means compares the subject walking information with the ideal walking information including the same hip joint angle as that of the subject walking information to evaluate the walking condition of the subject.

## Description

### TECHNICAL FIELD

The present invention relates to a walking condition evaluation system evaluating a walking condition of a subject.

### BACKGROUND ART

Recently, attention has been focused on a relationship between a walking condition and a health condition of a person, and in order to clarify the relationship, various technologies for evaluating the walking condition have been proposed. For example, there is a technology of defining and evaluating a walking condition, on the basis of the angle of a hip joint of the like (refer to Patent Document 1).

### CITATION LIST

### PATENT DOCUMENT

Patent Document 1: JP 2007-236663 A

### SUMMARY OF THE INVENTION

### PROBLEM TO BE SOLVED BY THE INVENTION

Walking for human beings involves a certain amount of burden on the body. Therefore, the quality of a walking condition greatly affects a health condition. In this case, it is desirable that a walking condition of a person can be evaluated from the viewpoint of whether it is suitable for maintaining the health condition. However, until now, the evaluation has not been performed from such a viewpoint.

The invention has been made in order to solve such problems, and an object thereof is to make it possible to evaluate a walking condition from the viewpoint of whether it is suitable for maintaining a health condition.

### MEANS FOR SOLVING PROBLEM

A first aspect for attaining the object described above is a walking condition evaluation system, including: an information acquisition means acquiring subject walking information relevant to a walking condition of a subject; a condition evaluation means comparing the walking information acquired by the information acquisition means with ideal walking information relevant to an ideal walking condition to evaluate the walking condition of the subject; and an evaluation output means outputting an evaluation result by the condition evaluation means, in which ideal walking information configured of a plurality of parameters including a hip joint angle in walking according to the ideal walking condition is prepared for each hip joint angle over a predetermined range, as the ideal walking information, the information acquisition means acquires a plurality of parameters including a hip joint angle in walking according to the subject, as the subject walking information, and the condition evaluation means compares the subject walking information with the ideal walking information including the same hip joint angle as that of the subject walking information to evaluate the walking condition of the subject.

When a person walks, the right and left legs motion to stretch backward and forward by mainly using a hip joint as a rotation center. Therefore, how other parts motion in cooperation with the hip joint greatly affects the quality of the walking condition.

For clarification, the applicant of this application has performed various types of analysis, on the basis of the walking condition of the subject. As a result thereof, the following tendency has been found. The tendency is that for the subject with a concern for a health condition, a burden on the joint is likely to increase, which indicates that the hip joint angle indicating the motion of the hip joint increases within a certain range as the health condition of the subject is excellent. From such a tendency, a suitable motion range of each part corresponding to the hip joint angle is an ideal walking condition, and it can be said that a burden on each joint (in particular, a hip joint, a knee joint, or the like) increases as deviating from the ideal walking condition.

As described above, the walking condition with a large burden is likely to cause disorderedness in each joint in the long term. Further, in a case where a pain and a sense of discomfort occurs as walking, a burden on other parts increases due to a motion to relieve the pain and the sense of discomfort, or the walking itself is avoided, and thus, even a muscle force necessary for life is not capable of being maintained, which increases a health concern. Therefore, in order to maintain an excellent health condition, it is effective to walk in an ideal walking condition in which a burden on the body is small.

Therefore, it is desirable to encourage the subject to walk in a walking condition in which a burden on the body is small. Therefore, first, it is required to grasp a difference between the walking condition of the subject and the ideal walking condition.

In response to such a demand, the walking condition evaluation system of the aspect described above compares the walking condition of the subject with the ideal walking condition, on the basis of the hip joint angle. Accordingly, from the viewpoint of whether it is suitable for maintaining the health condition, it is possible to evaluate the walking condition. By outputting the evaluation result of the walking condition evaluated as described above, it is possible to grasp a difference between the walking condition of the subject and the ideal walking condition. As a result thereof, it is possible to encourage the subject to walk in the ideal walking condition.

Note that as the walking information, it is considered to use some parameters suitable for evaluating the walking condition, in addition to the hip joint angle. As the parameter suitable for evaluating the walking condition, for example, it is considered to use a stride, a knee joint angle, or the like.

In this case, the aspect described above may be a second aspect described below. In the second aspect, the ideal walking information is configured of a plurality of parameters including the hip joint angle, and a stride and a knee joint angle at the hip joint angle, and the information acquisition means acquires the hip joint angle in walking according to the subject, and the stride and the knee joint angle at the hip joint angle, as the subject walking information.

The walking condition evaluation system of this aspect compares the walking condition of the subject with the ideal walking condition, on the basis of the hip joint angle, and the stride and the knee joint angle, and thus, is capable of evaluating the walking condition.

This aspect may be a third aspect described below. In the third aspect, the ideal walking information is configured of a plurality of parameters including the hip joint angle, a value represented on percentage by dividing the stride at the hip joint angle by a body length or a leg length, as the stride at the hip joint angle, and the knee joint angle at the hip joint angle, the information acquisition means acquires the hip joint angle in walking according to the subject, and the stride, the body length or the leg length, and the knee joint angle at the hip joint angle, as the subject walking information, and the condition evaluation means uses the stride represented on percentage by dividing the stride by the body length or the leg length, as the stride in the subject walking information, when evaluating the walking condition of the subject.

The walking condition evaluation system of this aspect uses the value represented on percentage by dividing the stride by the body length or the leg length, as the stride. Accordingly, it is possible to suppress the influence of the body length or the leg length, and more suitably evaluate the walking condition.

In addition, a walking condition evaluation system of a fourth aspect for attaining the object described above, includes: an information acquisition means acquiring walking information when a subject walks; a state determination means determining to which walking condition among a plurality of patterns of walking conditions set in advance a walking condition defined by the walking information acquired by the information acquisition means corresponds; an evaluation specifying means specifying evaluation information corresponding to the walking condition determined by the state determination means, among evaluation information associated with each of the plurality of patterns of the walking conditions, as information including evaluation of the walking condition and advice according to the evaluation; and an evaluation output means outputting the evaluation information specified by the evaluation specifying means.

The walking condition evaluation system of the aspect described above classifies the walking condition into the plurality of patterns, in accordance with the health condition, and determines and evaluates which pattern the walking condition of the subject has. Accordingly, it is possible to suppress a processing load required for evaluation.

In addition, the aspect described above may be a fifth aspect described below.

In the fifth aspect, an image acquisition means acquiring image data obtained by capturing a state of the subject walking is provided, and the information acquisition means acquires walking information including a stride and a hip joint angle of the subject, on the basis of the image data acquired by the image acquisition means.

In daily life, in a situation such as deterioration in the health condition, aging, and the middle-aged, there is a lack of exercise for encouraging the extension of the hip joint, and thus, the hip joint angle in walking decreases. The applicant of this application has focused on such a tendency and has studied which parameter is to be used to define the walking condition centering on the hip joint angle. As a result thereof, the availability of defining the walking condition by the hip joint angle and the stride has been found.

There is a tendency that a relationship between the hip joint angle in walking and the stride for each of a plurality of subjects is different between subjects with no problems in a health condition and subjects with some problems in a health condition. The availability described above indicates that the evaluation of the walking condition is available on the basis of such a tendency. The applicant of this application has conceived the configuration as described above with originality and ingenuity to use such a tendency in the evaluation of the walking condition.

In order to evaluate the ability to walk, the walking condition can be defined by various parameters including not only the hip joint angle but also the knee joint angle and the like. On the other hand, in a case where there are various parameters, the processing load required for evaluation, or the like increases. Therefore, as described above, it is preferable that the walking condition can be defined and evaluated by the hip joint angle and the stride, from the viewpoint of reducing a processing load required for evaluation.

### EFFECT OF THE INVENTION

Therefore, the walking condition evaluation system of the aspect described above acquires the walking information including the stride and the hip joint angle, and thus, is capable of suppressing the processing load required for evaluation and effectively evaluating the walking condition.

### BRIEF DESCRIPTION OF DRAWINGS

**Fig.** 1 is a block diagram illustrating a configuration of a walking condition evaluation system in the present disclosure;
**Fig.** 2 is a schematic view illustrating a state of capturing a subject with a camera in the disclosure;
**Fig.** 3 is a flowchart illustrating a procedure of evaluation output processing;
**Fig.** 4 is a diagram (1) in which a subject included in an image is modeled as a skeleton;
**Fig.** 5 is a diagram (1) illustrating a page for outputting evaluation information;
**Fig.** 6 is a flowchart (1) illustrating a procedure of condition evaluation processing;
**Fig.** 7 is a diagram illustrating a pattern of a walking condition;
**Fig.** 8 is a diagram (2) in which a subject included in an image is modeled as a skeleton;
**Fig.** 9 is a diagram (2) illustrating a page for outputting evaluation information; and
Fig. 10 is a flowchart (2) illustrating a procedure of condition evaluation processing.

### MODE(S) FOR CARRYING OUT THE INVENTION

Hereinafter, embodiments of the invention will be described together with the drawings.

### (1) Overall Configuration

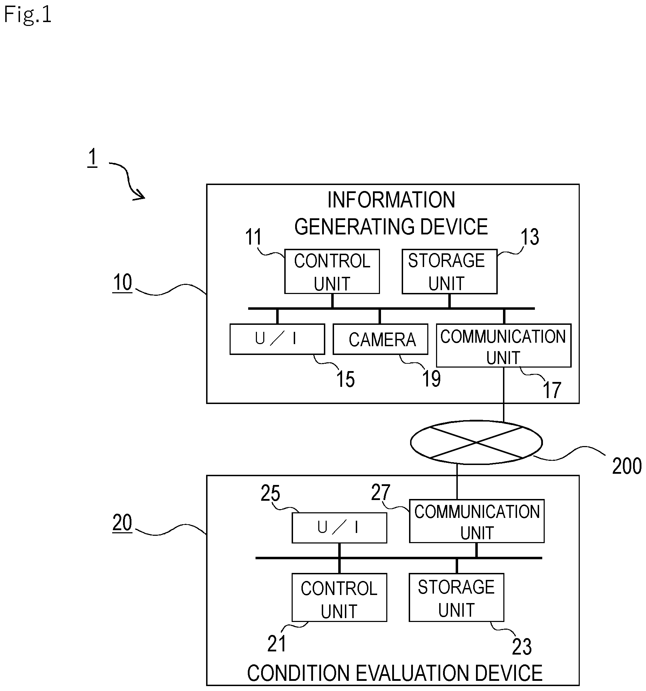

As illustrated in Fig. 1, a walking condition evaluation system 1 includes an information generating device 10 and a condition evaluation device 20. The information generating device 10 generates walking information relevant to the walking of a subject. In addition, the condition evaluation device 20 evaluates the walking condition of the subject.

The information generating device 10 includes a control unit 11, a storage unit 13, a user interface (U/I) 15, a communication unit 17, and a camera 19. The control unit 11 controls the operation of the entire information generating device 10. The storage unit 13 stores various types of data. The user interface 15 performs an interaction with a user. The communication unit 17 controls communication with the condition evaluation device 20. The camera 19 is installed to capture the state of the subject walking.

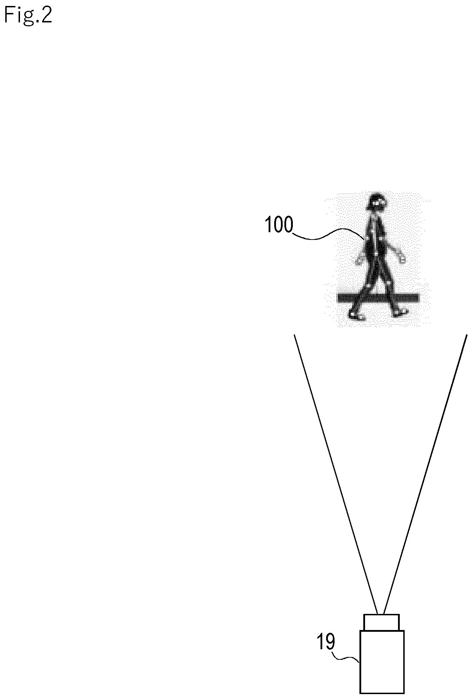

Among them, the camera 19, as illustrated in Fig. 2, is installed at a position where the state of a subject 100 walking can be captured from the side. Then, the camera 19 captures the state of the subject 100 walking to generate image data. Such image data indicates each of a plurality of images continuous in chronological order.

The condition evaluation device 20 includes a control unit 21, a user interface (U/I) 25, and a communication unit 27. The control unit 21 controls the operation of the entire condition evaluation device 20. The storage unit 23 stores various types of data. The user interface 25 performs an interaction with the user. The communication unit 27 controls communication with the information generating device 10.

Note that in this embodiment, the information generating device 10 and the condition evaluation device 20 are connected through the internet 200 such that communication is available. The information generating device 10 and the condition evaluation device 20 may be connected directly such that communication is available.

### (2) First Embodiment

Here, as a first embodiment, various processing procedures by the walking condition evaluation system 1 with the configuration described above will be described.

### (2-1) Evaluation Output Processing

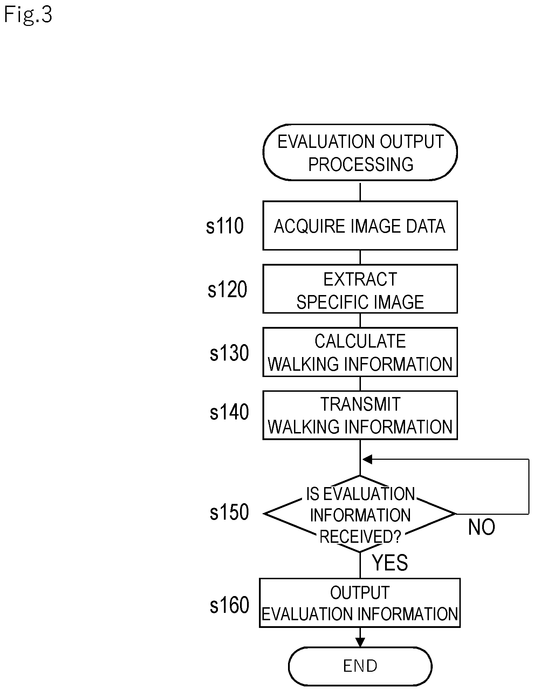

First, a processing procedure of evaluation output processing executed by the control unit 11 of the information generating device 10 will be described on the basis of Fig. 3. Such evaluation output processing is executed in accordance with a program stored in the storage unit 13. Such evaluation output processing is activated when receiving a command for activating the evaluation output processing through the user interface 15.

In such evaluation output processing, first, the image data is acquired from the camera 19 (s110). Here, in accordance with a timing when the subject crosses the capturing range of the camera 19, commands for starting and ending the capturing are sequentially issued from the control unit 11 to the camera 19. Then, the image data indicating the plurality of continuous images captured in chronological order between such commands is acquired.

Here, the commands to the camera 19 for starting and ending the capturing may be artificially issued at a timing when a command from the user is received through the user interface 15. Such commands may be automatically issued on the basis of a change in the image in the capturing range or a detection result of a sensor that is not illustrated.

Next, a specific image is extracted from the image data acquired in s110 described above (s120). Here, first, each subject reflected in each image indicated by the image data is modeled as a skeleton. After that, in such a skeleton model, a heel-off image and a heel-contact image are extracted on the basis of the position of the heel of a leg to be an observation target (a leg on the front side in a leg image; hereinafter, also referred to as an "observed leg").

The "heel-off image" is an image in a heel-off state in which the heel on a hip joint extension side among the observed legs of the subject while walking is separated from the ground surface. In addition, the "heel-contact image" is an image in a heel-contact state in which the heel on a hip joint flexion side among the observed legs of the subject during walking is in contact with the ground surface. The image extracted in s120 is any one set of image data indicating each of the heel-off image and the heel-contact image of the observed leg, which are in tandem in chronological order.

Note that images in which the heel of the observed leg is at positions corresponding to the heel-off state and the heel-contact state are extracted from an image group captured by the camera 19, as the heel-off image and the heel-contact image. However, a sensor (for example, a vibration sensor, an acceleration sensor, or the like provided on the subject or the road surface) may sense that the subject is in the heel-off state and the heel-contact state during walking, and may extract images at the sensed timing, as the heel-off image and the heel-contact image.

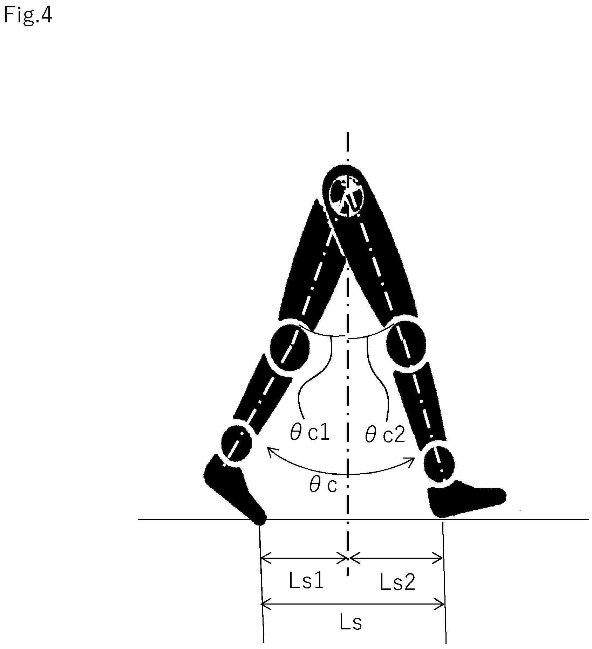

Next, the walking information of the subject is calculated on the basis of each of the images extracted in s120 described above (s130). Here, as illustrated in Fig. 4, a hip joint angle θc, a stride Ls, a body length Lt, and the like of the subject are calculated. The hip joint angle θc, the stride Ls, the body length Lt, and the like are calculated from each of the heel-off image and the heel-contact image extracted in s120 described above. Note that Fig. 4 illustrates a state in which the observed leg in the heel-contact state and the observed leg in the heel-off state are superimposed such that the rotation centers of the hip joints are coincident with each other.

Here, the hip joint angle θc is calculated as an angle (θc = θc1 + θc2) obtained by adding a hip joint extension angle θc1 of the leg in the heel-off state in the heel-off image and a hip joint flexion angle θc2 of the leg in the heel-contact state in the heel-contact image together. The hip joint extension angles θc1 and θc2 are an angle between an axis from the hip joint to the knee and a vertical direction.

In addition, the stride Ls is calculated as a distance (Ls = Ls1 + Ls2) obtained by adding a heel-off distance Ls1 and a heel-contact distance Ls2 together. The heel-off distance Ls1 is a distance from the position of the toe (in this embodiment, the tiptoe) in the heel-off state in the subject to a position immediately below the hip joint forward in a traveling direction. The heel-contact distance Ls2 is a distance from the position of the toe (in this embodiment, the tiptoe) in the heel-contact state to a position of the hip joint immediately below backward in the traveling direction.

Here, the stride Ls is calculated by focusing on the heel-off image and the heel-contact image of the observed leg, which are in tandem in chronological order, that is, the position of the toe in each state of the observed leg from the heel-off state to the heel-contact state. However, the stride Ls may be calculated by focusing on the position of the toe of the observed leg and the position of the toe of the opposite leg (hereinafter, referred to as a "non-observed leg"). Specifically, it is considered to perform the same calculation as described above, on the basis of the toe of the observed leg in either the heel-off state or the heel-contact state, and the toe of the non-observed leg in the other state in an image close to the image of the above state in chronological order.

In addition, the body length Lt is calculated as a distance (Lt = Ll + Lb) obtained by adding an average distance Ll of the length of each leg of the subject in each of the heel-off image and the heel-contact image and a distance Lb from the hip joint to the head of the subject in each of the images together.

Next, the walking information calculated in s130 described above is transmitted to the condition evaluation device 20 (s140). The condition evaluation device 20 receives the walking information to execute condition evaluation processing described below, and returns evaluation information corresponding to the walking information.

When the walking information is transmitted as described above, a stand-by state is set until the evaluation information from the condition evaluation device 20 is returned (s150: NO). After that, when the evaluation information is returned (s150: YES), the evaluation information is output (s160), and then, this evaluation output processing is ended.

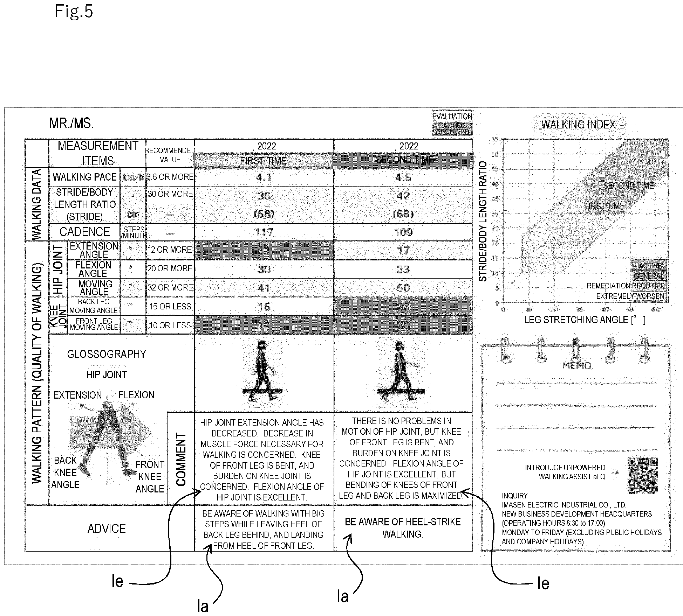

In s160, first, as illustrated in Fig. 5, page data is generated in which evaluation contents Ie and advice Ia included in the evaluation information are arranged in a predetermined format. Then, the page data is output through the user interface 15 (for example, printed on paper, displayed on a display, or the like).

Note that in the evaluation output processing of this embodiment, after the heel-off image and the heel-contact image are extracted, on the basis of the heel-off image and the heel-contact image, the walking information of the subject is calculated. However, after each of the images is extracted, whether such images are suitable for the calculation of the walking information may be checked. Then, in a case where the image is not appropriate, processing of acquiring again the image data may be performed, or the evaluation output processing itself may be ended.

### (2-2) Condition Evaluation Processing

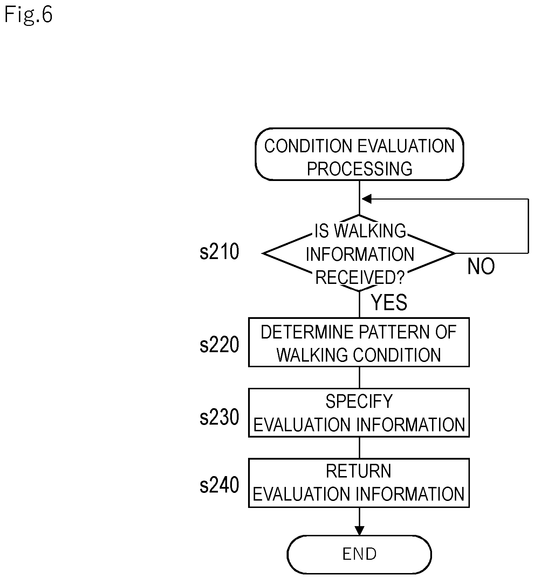

Subsequently, a processing procedure of condition evaluation processing executed by the control unit 21 of the condition evaluation device 20 will be described on the basis of Fig. 6. Such condition evaluation processing is executed in accordance with a program stored in the storage unit 23, after the condition evaluation device 20 is activated.

In such condition evaluation processing, first, a stand-by state is set until the walking information is received from the information generating device 10 (s210: NO). After that, when the walking information is received from the information generating device 10 (s210: YES), the pattern of the walking condition defined by the walking information is determined (s220). In s220, to which walking condition among a plurality of patterns of walking conditions set in advance the walking condition defined by the walking information corresponds is determined.

Here, as the walking condition defined by the walking information received in s210, to which pattern of walking condition the walking condition defined by at least a stride rate (Ls/Lt) obtained by dividing the stride Ls of the subject by the body length Lt and the hip joint angle θc corresponds is determined.

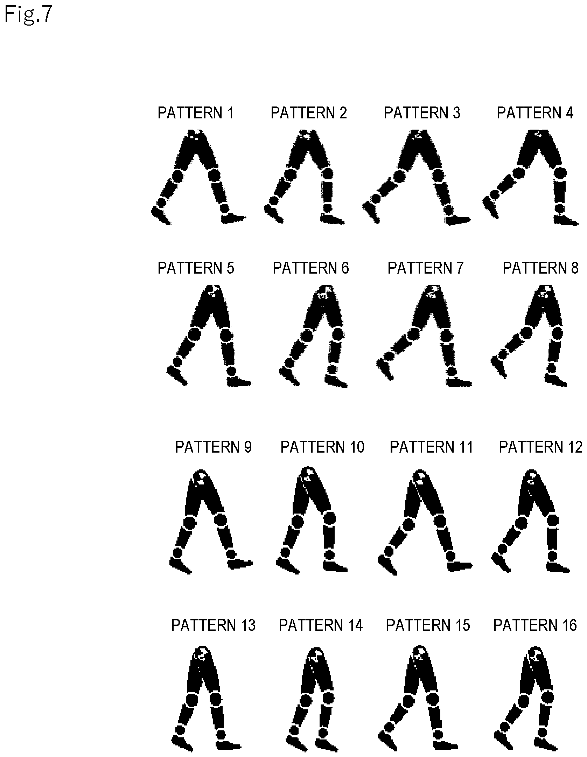

The plurality of patterns of walking conditions set in advance are registered in a database stored in the storage unit 23. Therefore, the determination is performed on the basis of the database. In this embodiment, as illustrated in Fig. 7, 16 patterns of walking conditions are registered in accordance with each parameter. However, walking conditions with patterns of less than or greater than 16 may be registered.

Next, the evaluation information corresponding to the walking condition determined in s220 described above is specified (s230). Here, among the evaluation information set in advance, evaluation information corresponding to the walking condition determined in s220 described above is specified. Such evaluation information is information including the evaluation contents as the walking condition and the advice based on the evaluation contents (refer to Fig. 5), and is stored in advance in the storage unit 23, as corresponding to each of the walking conditions. Therefore, the corresponding evaluation information is specified therefrom.

Then, the evaluation information specified in s230 described above is returned to the condition evaluation device 20 (s240), and then, this condition evaluation processing is ended.

### (2-3) Modification Example

The first embodiment of the invention has been described above. However, the invention is not limited to the embodiment described above, and it is obvious that various forms can be taken within the technical scope of the invention.

For example, in the embodiment described above, a configuration has been exemplified in which the walking information is acquired from the image data obtained by the capturing of the camera 19. However, the walking information may be configured to be acquired by a method other than the capturing of the camera 19. In addition, the walking information may be configured to be acquired from the outside the information generating device 10 through the communication unit 17 or the like.

In addition, in the embodiment described above, a configuration has been exemplified in which the stride rate obtained by dividing the stride Ls by the body length Lt is used to determine to which of the plurality of patterns of walking conditions the walking condition corresponds (s220 of condition evaluation processing). However, in such determination, the stride Ls itself may be used, or a value obtained by dividing the stride Ls by other parameters such as the length of the leg (hereinafter, referred to as a "leg length")) or a lower body length. Here, in a case where the stride Ls itself is used, in order to suppress the influence of a factor such as race on a ratio of leg length to the body length, a value multiplied by a predetermined coefficient according to the factor may be used.

In addition, in the embodiment described above, a configuration has been exemplified in which the evaluation information is output through the user interface 15 of the information generating device 10. However, the evaluation information may be output through the user interface 25 of the condition evaluation device 2, or may be transmitted from the condition evaluation device 2 to the external device through the communication unit 27.

In addition, in the embodiment described above, a configuration has been exemplified in which the walking condition evaluation system 1 includes two devices of the information generating device 10 and the condition evaluation device 20. However, such a walking condition evaluation system 1 may be configured of one device including all the constituents of each of the devices. In addition, three or more devices connected to each other such that communication is available may include all the constituents of each of the devices in a distributed manner.

### (2-4) Function Effect

In the walking condition evaluation system 1 of the embodiment described above, the walking condition is classified into the plurality of patterns, in accordance with the health condition, and which pattern the walking condition of the subject has is determined and evaluated. Accordingly, it is possible to suppress a processing load required for evaluation.

In daily life, in a situation such as deterioration in the health condition, aging, and the middle-aged, there is a lack of exercise for encouraging the extension of the hip joint, and thus, the hip joint angle in walking decreases. The applicant of this application has focused on such a tendency and has studied which parameter is to be used to define the walking condition centering on the hip joint angle. As a result thereof, the availability of defining the walking condition by the hip joint angle and the stride has been found.

There is a tendency that a relationship between the hip joint angle in walking and the stride for each of a plurality of subjects is different between subjects with no problems in a health condition and subjects with some problems in a health condition. The availability described above indicates that the evaluation of the walking condition is available on the basis of such a tendency. The applicant of this application has conceived the configuration as described above with originality and ingenuity to use such a tendency in the evaluation of the walking condition.

In order to evaluate the ability to walk, the walking condition can be defined by various parameters including not only the hip joint angle but also the knee joint angle and the like. On the other hand, in a case where there are various parameters, the processing load required for evaluation, or the like increases. Therefore, as described above, it is preferable that the walking condition can be defined and evaluated by two types of parameters, from the viewpoint of reducing the processing load required for evaluation.

Therefore, the walking condition evaluation system 1 of the embodiment described above acquires the walking information including the stride and the hip joint angle, and thus, is capable of suppressing the processing load required for evaluation and effectively evaluating the walking condition.

### (3) Second Embodiment

Subsequently, as a second embodiment, various processing procedures by the walking condition evaluation system 1 with the configuration described above will be described.

### (3-1) Evaluation Output Processing

First, a processing procedure of evaluation output processing in this embodiment will be described.

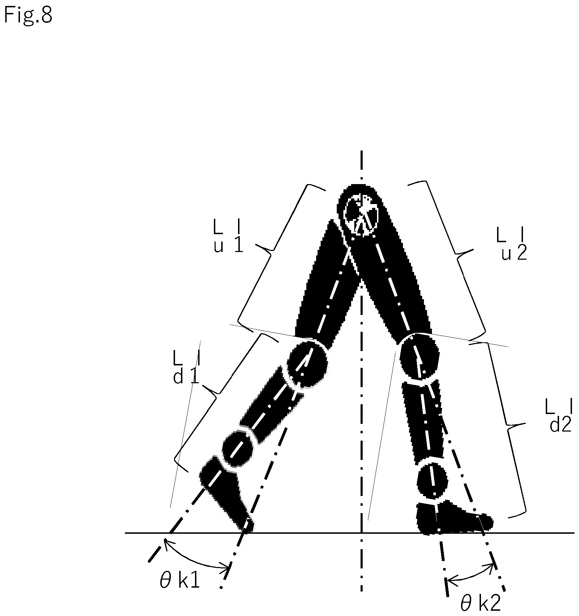

In such evaluation output processing, as with the first embodiment, s110 to s120 are performed, and then, the walking information of the subject is calculated (s130). Here, the hip joint angle θc, the stride Ls, the body length Lt, the leg length Ll, a knee joint angle θk, and the like of the subject are calculated from each of the heel-off image and the heel-contact image extracted in s120 described above (refer to Fig. 4 and Fig. 8).

As the knee joint angle θk, two parameters of a knee joint extension angle θk1 and a knee joint flexion angle θk2 are calculated. The knee joint extension angle θk1 is an angle formed by the axis of the thigh of the leg in the heel-off state (that is, the leg on the extension side; the same applies below) and the axis of the lower thigh. The knee joint flexion angle θk2 is an angle formed by the axis of the thigh of the leg in the heel-contact state (that is, the leg on the flexion side; the same applies below) and the axis of the lower thigh. Here, an angle formed by a straight line obtained by extending the axis of the thigh toward the lower thigh and the axis of the lower thigh is set as the knee joint angle. However, an angle between the thigh and the lower thigh (that is, the angle formed by the axis of the thigh and the axis of the lower thigh) may be set as the knee joint angle θk.

In addition, the leg length Ll is calculated as an average value ((Ll1 + L12)/2) of a sum Ll1 of a length Llu1 of the thigh of the leg in the heel-off state and a length Lld1 of the lower thigh, and a sum Ll2 of a length Llu2 of the thigh of the leg in the heel-contact state and a length Lld2 of the lower thigh.

Next, the walking information (hereinafter, referred to as "subject walking information") calculated in s130 described above is transmitted to the condition evaluation device 20 (s140). When the walking information is transmitted as described above, a stand-by state is set until the evaluation information is returned from the condition evaluation device 20 (s150: NO).

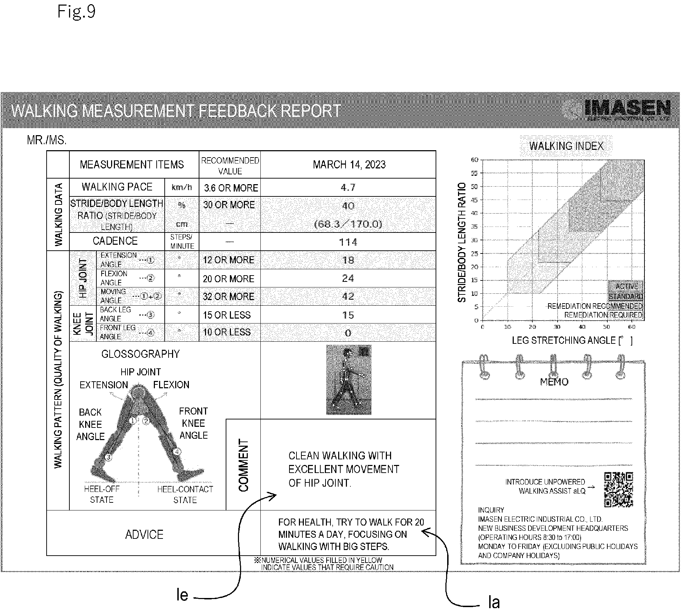

After that, when the evaluation information is returned (s150: YES), the evaluation information is output (s160), and then, this evaluation output processing is ended. In s160, as illustrated in Fig. 9, the page data is generated in which the evaluation contents Ie and the advice Ia included in the evaluation information are arranged in a predetermined format. Then, the page data is output through the user interface 15.

### (3-2) Condition Evaluation Processing

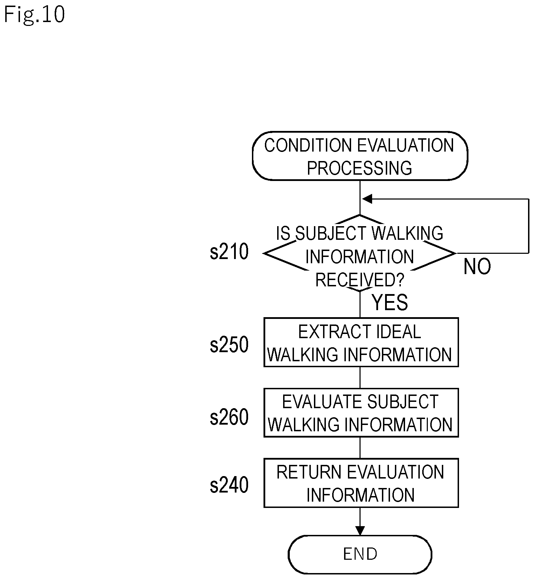

Subsequently, a processing procedure of condition evaluation processing in this embodiment will be described on the basis of Fig. 10.

In such condition evaluation processing, first, a stand-by state is set until the subject walking information is received from the information generating device 10 (s210: NO). After that, when the subject walking information is received (s210: YES), walking information indicating an ideal walking condition (hereinafter, also referred to as "ideal walking information") is extracted on the basis of the walking condition defined by the subject walking information (s250). Here, among a plurality of types of ideal walking information prepared in advance, ideal walking information with the same hip joint angle (coincident in a predetermined error range) as that of the subject walking information received in 210 described above is extracted.

In this embodiment, the ideal walking information configured of a plurality of parameters including the hip joint angle in walking according to the ideal walking condition, and a stride and a knee joint angle at the hip joint angle is prepared in advance for each hip joint angle over a predetermined range. Here, the ideal walking information is stored in the storage unit 23. The ideal walking condition described above is a walking condition in which a burden on the body is small. Then, in s250, the corresponding ideal walking information is extracted from the ideal walking information prepared in advance.

Note that in walking, it is desirable to bend the knee joint moderately in order to reduce the impact when the heel is in contact with the ground surface. However, in a case where the knee joint is fully extended, a large burden occurs on the knee joint. On the other hand, in a case where the knee joint is excessively bent, the joint does not move sufficiently, and in some cases, a large burden may occur on other joints such as the lower back and the hip joint. In this embodiment, from such a viewpoint, the ideal walking condition is defined. Specifically, the ideal walking condition is defined on the basis of a triangle in which the hip joint angle is set as the apex angle, and an ideal stride at the hip joint angle is set as the base. The ideal walking condition is a walking condition in which the knee joint angle between both legs that are two sides interposing the apex of the triangle therebetween is an ideal knee joint angle. As the ideal knee joint angle, the knee joint extension angle θk1 is 10 degrees or less, and the knee joint flexion angle θk2 is 15 degrees or less.

Next, the subject walking information received in s210 described above is evaluated on the basis of the ideal walking information extracted in s250 described above (s260). Here, by comparing each parameter in both types of walking information (the ideal walking information and the subject walking information), the size of the hip joint angle θc, a difference between the stride Ls and the ideal stride, and a difference between the knee joint angle θk and the ideal knee joint angle are evaluated. After that, the evaluation information according to a combination of such evaluation values is specified as with the first embodiment.

Then, evaluation information indicating an evaluation result by s250 described above is returned to the condition evaluation device 20 (s240), and then, this condition evaluation processing is ended.

### (3-3) Modification Example

The second embodiment of the invention has been described above. However, the invention is not limited to the embodiment described above, and it is obvious that various forms can be taken within the technical scope of the invention.

For example, in the embodiment described above, a configuration has been exemplified in which the walking condition is evaluated by using the stride Ls included in the walking information. As the stride Ls, a value represented on percentage by dividing the stride Ls by the body length Lt or the leg length Ll may be used. In this case, for the ideal walking information, the stride may be represented on percentage, and in the condition evaluation processing, the stride Ls of the subject walking information may be converted into a percentage and evaluated.

In addition, in the embodiment described above, in the evaluation output processing, a configuration has been exemplified in which the knee joint extension angle θk1 and the knee joint flexion angle θk2 are calculated as the knee joint angle θk. However, as the knee joint angle θk, for example, as the calculated average value of the knee joint extension angle θk1 and the knee joint flexion angle θk2 may be used, or one of the hip joint angles may be selected and used. In this case, for the ideal walking information, evaluation may be performed on the basis of the knee joint angle θk calculated or selected as the same average value.

### (3-4) Function Effect

As described above, the walking condition with a large burden is likely to cause disorderedness in each joint in the long term. This is because in a case where a pain and a sense of discomfort occurs as walking, a burden on other parts increases due to a motion to relieve the pain and the sense of discomfort, and the walking itself is avoided, and thus, even a muscle force necessary for life is not capable of being maintained, which increases a health concern. Therefore, in order to maintain an excellent health condition, it is effective to walk in the ideal walking condition in which a burden on the body is small.

Therefore, it is desirable to encourage the subject to walk in a walking condition in which a burden on the body is small. Therefore, first, it is required to grasp a difference between the walking condition of the subject and the ideal walking condition.

In response to such a demand, the walking condition evaluation system of the embodiment described above compares the walking condition of the subject with the ideal walking condition, on the basis of the hip joint angle θc**.** Accordingly, from the viewpoint of whether it is suitable for maintaining the health condition, it is possible to evaluate the walking condition. By outputting the evaluation result of the walking condition evaluated as described above, it is possible to grasp a difference between the walking condition of the subject and the ideal walking condition. As a result thereof, it is possible to encourage the subject to walk in the ideal walking condition.

In addition, the walking condition evaluation system 1 of the embodiment described above compares the walking condition of the subject with the ideal walking condition, on the basis of the hip joint angle θc**,** the stride Ls, and the knee joint angle θk. Accordingly, it is possible to evaluate the walking condition.

In addition, in the walking condition evaluation system 1 of the embodiment described above, in a case where the stride rate (the value represented on percentage by dividing the stride Ls by the body length Lt or the leg length Ll) is used for evaluation, it is possible to suppress the influence of the body length Lt or the leg length Ll, and more suitably evaluate the walking condition.

### (4) Correspondence Relationship with Present Invention

In the embodiment described above, in the evaluation output processing, s110 is an image acquisition means, s120 is an image extraction means, s130 is a first calculation means and a second calculation means, and s160 is an evaluation output means. In addition, in the condition evaluation processing, s210 is an information acquisition means, and s230 and s260 are a condition evaluation means.

### EXPLANATIONS OF LETTERS OR NUMERALS

- 1: WALKING CONDITION EVALUATION SYSTEM
- 10: INFORMATION GENERATING DEVICE
- 11: CONTROL UNIT
- 13: STORAGE UNIT
- 15: USER INTERFACE
- 17: COMMUNICATION UNIT
- 19: CAMERA
- 20: CONDITION EVALUATION DEVICE
- 21: CONTROL UNIT
- 23: STORAGE UNIT
- 27: COMMUNICATION UNIT
- 100: SUBJECT
- 200: INTERNET

## Claims

1. A walking condition evaluation system, comprising:
an information acquisition means;
a condition evaluation means; and
an evaluation output means,
wherein the information acquisition means acquires subject walking information relevant to a walking condition of a subject,
the condition evaluation means compares the walking information acquired by the information acquisition means with ideal walking information relevant to an ideal walking condition to evaluate the walking condition of the subject,
the evaluation output means outputs an evaluation result by the condition evaluation means,
ideal walking information configured of a plurality of parameters including a hip joint angle in walking according to the ideal walking condition is prepared for each hip joint angle over a predetermined range, as the ideal walking information,
the information acquisition means acquires a plurality of parameters including a hip joint angle in walking according to the subject, as the subject walking information, and
the condition evaluation means compares the subject walking information with the ideal walking information including the same hip joint angle as that of the subject walking information to evaluate the walking condition of the subject.

2. The walking condition evaluation system according to claim 1,
wherein the ideal walking information is configured of a plurality of parameters including the hip joint angle, and a stride and a knee joint angle at the hip joint angle, and
the information acquisition means acquires the hip joint angle in walking according to the subject, and the stride and the knee joint angle at the hip joint angle, as the subject walking information.

3. The walking condition evaluation system according to claim 1,
wherein the ideal walking information is configured of a plurality of parameters including the hip joint angle, a value represented on percentage by dividing a stride at the hip joint angle by a body length or a leg length, as the stride at the hip joint angle, and a knee joint angle at the hip joint angle,
the information acquisition means acquires the hip joint angle in walking according to the subject, and the stride, the body length or the leg length, and the knee joint angle at the hip joint angle, as the subject walking information, and
the condition evaluation means uses a stride represented on percentage by dividing the stride by the body length or the leg length, as the stride in the subject walking information, when evaluating the walking condition of the subject.

4. The walking condition evaluation system according to any one of claims 1 to 3,
wherein the condition evaluation means compares the subject walking information with the ideal walking information including the same hip joint angle as that of the subject walking information to evaluate a size of the hip joint angle and evaluate a difference in each of the parameters excluding the hip joint angle.

5. A program for causing a computer to function as each of the means according to claim 1.
